# EUROPEAN PATENT APPLICATION

(11) **EP 4 620 486 A1**
(43) Date of publication of application: **24.09.2025**
(21) Application number: 25164482.9
(22) Date of filing: 18.03.2025
(51) Int. Cl.: A61K 47/54, A61P 3/04

(54) **CONJUGATES OF G-PROTEIN COUPLED RECEPTOR AGONISTS AND ANTAGONISTS**

(30) Priority: 19.03.2024 US 202463567051 P
(71) Applicant: Bioxel, Inc., Cambridge, MA 02139 (US)
(72) Inventor: GLEASON, Patrick R., Cambridge, 02139 (US)
(74) Representative: Pearl Cohen Zedek Latzer Baratz UK LLP

(57) **Abstract**

Compounds comprising at least one GPCR agonist and/or antagonist and at least one oral bioavailability enhancing moiety and/or protein binding enhancing moiety, for treatment of GPCR agonist or antagonist responsive diseases and conditions via the oral route of administration, among other routes.

## Description

### CROSS-REFERENCE TO RELATED APPLICATIONS

This application claims the benefit of U.S. Provisional Application No. 63/567,051, filed March 19, 2024, which is hereby incorporated by reference in its entirety.

### INCORPORATION BY REFERENCE OF SEQUENCE LISTING

The instant application contains a Sequence Listing conforming the rules of WIPO Standard ST.26 which has been submitted electronically in XML format and is hereby incorporated by reference in its entirety. The XML copy, created on March 15, 2025, is named P-633929-US_SQL_15MAR25.xml, and is 67,037 bytes in size.

### FIELD OF THE INVENTION

The present invention relates to compounds comprising at least one GPCR agonist and/or antagonist and at least one oral bioavailability enhancing moiety and/or protein binding enhancing moiety, as well as pharmaceutical compositions comprising said compounds. The present invention relates to use of the compounds comprising at least one GPCR agonist and/or antagonist and at least one oral bioavailability enhancing moiety and/or protein binding enhancing moiety for treatment of GPCR agonist or antagonist responsive diseases and conditions via various routes of administration, in particular, by the oral route of administration. The present invention further relates to treating a GPCR agonist and/or antagonist responsive condition or disease by administering to a subject in need thereof the compound comprising at least one GPCR agonist and/or antagonist and at least one oral bioavailability enhancing moiety and/or protein binding enhancing moiety or administering a pharmaceutical composition comprising said compound.

### BACKGROUND OF THE INVENTION

Type 2 diabetes affects about 1 in 10 Americans, and an estimated 537 million adults worldwide. WHO statistics show that as of 2022, 1 in 8 people were affected by obesity, this number having more than doubles since 1990 and quadrupled in adolescents, and that 2.5 billion adults, 18 years and older were overweight, of which 890 million were obese and that 37 million children under the age of 5 were overweight. Obesity increases an individual's risk of developing type 2 diabetes, heart disease, and certain cancers, in addition to adversely affecting bone health and reproduction, as well as quality of life. Obesity and insulin resistance, i.e., cells become less responsive to insulin, frequently coexist; insulin resistance is a characteristic of the etiology of type 2 diabetes, and is linked to various pathophysiologic complications or conditions, including hypertension, hyperlipidemia, atherosclerosis, and polycystic ovarian disease. Osteoporosis, a loss of bone mass and change on bone tissue structure, occurs in post-menopausal women, men with low testosterone, in individuals having a diet deficient in calcium and vitamin D or protein, in certain medical conditions, including but not limited to endocrine and hormonal diseases, gastrointestinal diseases, rheumatoid arthritis, certain types of cancer, HIV/AIDS, and anorexia nervosa, as well as from drugs, including but not limited to glucocorticoids and adrenocorticotropic hormone, some cancer medications, proton pump inhibitors, antidepressants, such as selective serotonin reuptake inhibitors, and thiazolidinediones (TZDs), a class of medications used to treat type II diabetes. Low levels of physical activity also contribute to osteoporosis.

Cardiovascular disease (CVD) can lead to a stroke and heart attack. Increases in the development of atherosclerosis and other metabolic disturbances, e.g., metabolic syndrome, diabetes mellitus, and hypertension, which that are highly prevalent in people with CVD, are associated with a diet high in calories, saturated fats, and sugars, as well as physical inactivity.

Migraine, a disabling primary headache disorder, is linked to several comorbidities, including depression and anxiety, sleep disorders, fatigue, cardiovascular risk factors, e.g., hypertension, diabetes, high cholesterol, and obesity, CVD and cerebrovascular diseases. High fat or high carbohydrate diets, and excessive caffeine have been reported to be associated with migraine.

Parkinson's disease (PD) and Alzheimer's disease (AD) are distinct neurodegenerative disorders that affect the brain and lead to a progressive decline in cognitive and motor functions. PD and AD affect almost 8 million people in the United States. Multiple sclerosis (MS) is a chronic central nervous system disease that is considered an autoimmune disorder, wherein the body's immune system attacks myelin that covers nerves. Almost 1 million people in the United States and 2.9 million people worldwide have MS. Traumatic brain injury (TBI) can be caused by a forceful bump, blow, or jolt to the head or body, or from an object entering the brain (a penetrating TBI). Physical symptoms of TBI include headache, dizziness, confusion, convulsion or seizures, blurred or double vision, unequal pupil size or dilation, nausea and vomiting, slurred speech weakness in the arms, legs or face and loss of balance. TBI also manifests cognitive or behavioral symptoms, as well as perception and sensation symptoms. Brain damage from TBI includes bleeding, swelling and nerve injuries.

Antagonists of GPCR receptors are useful for blocking hormone activity, manipulation of the reproductive endocrine axis, and treatment of cancer.

Oral administration remains a widespread route for drug delivery because of pain avoidance, ease of ingestion, patient compliance and versatility of drug candidates. Nevertheless, the efficacy and therapeutic effect of orally administered drugs may be constrained by poor bioavailability of oral formulations. Aspects that contribute to low oral bioavailability include physiological factors, high gastric emptying time, the effect of food, intestinal barrier and enzymatic degradation of drugs. One of the key factors responsible for poor bioavailability of oral drug formulations is wide-ranging metabolism of drugs prior to reaching the systemic circulation, which is known as first-pass metabolism. First-pass metabolism encompasses drug absorption by the gastrointestinal tract and drug metabolism in the liver, prior to arriving to systemic circulation, thereby causing low drug plasma concentrations to reach its intended biological destination. Another consideration for effective therapeutic efficacy is the protein binding of a drug. Certain drugs demonstrate high protein binding, which increases the half-life, e.g., by rendering the drug unavailable to metabolism by the liver or making it unable to pass through glomerular capillaries in the kidneys.

Accordingly, there remains a critical need for developing improved compositions and methods for treatment of the above-described diseases and conditions, especially compositions having enhanced oral bioavailability.

### SUMMARY

In one aspect, a compound is provided comprising at least one G-protein coupled receptor (GPCR) agonist and/or antagonist covalently bound to at least one oral bioavailability enhancing moiety and/or protein binding enhancing moiety. In some embodiments, the at least one GPCR agonist and/or antagonist is a GLP-1 agonist or antagonist, a GIP agonist or antagonist, or a CRLR agonist or antagonist. In some embodiments the at least one or all GPCR agonists and/or antagonists in the compound are peptides. In some embodiments, the at least one GPCR agonist and/or antagonist is a GLP-1 agonist covalently bound to a GIP agonist. In some embodiments, at least one GPCR agonist and/or antagonist is a GLP-1 agonist covalently bound to a CRLR agonist.

In some embodiments, each of the at least one oral bioavailability enhancing moiety and/or protein binding enhancing moiety is independently bound to an N-terminal amino acid residue, to a C-terminal amino acid residue, or to any non-terminal amino acid residue of the at least one GPCR agonist and/or antagonist, or introduced or replaced therein, or bound to any other component thereof. In some embodiments, the non-terminal amino acid residue is a lysine.

In some embodiments, the at least one oral bioavailability enhancing moiety and/or protein binding enhancing moiety comprises a bile salt or bile acid. In some embodiments, the bile salt or bile acid comprises lithocholic acid, cholic acid, chenodeoxycholic acid, deoxycholic acid, ursodeoxycholic acid, or 7-ketolithocholic acid.

In some embodiments, the oral bioavailability enhancing moiety is an enhancer of transcellular transport, such as a fatty acid or a derivative thereof, e.g., oleic acid or an acylcarnitine; or a lipophilic compound, e.g., a lipophilic agent enhancing membrane permeability. In some embodiments, the oral bioavailability enhancing moiety enhances paracellular transport, such as a medium chain fatty acid, e.g., capric acid; or a surfactant e.g., sodium caprate or sodium lauryl sulfate; or a calcium chelator, e.g., EDTA or DOTA; or a bile salt such as those described herein, that modulate tight junctions.

In some embodiments, the at least one GPCR agonist and/or antagonist, or any one or all GPCR agonists and/or antagonists in the compound, is or are peptides. In some embodiments, all GPCR agonists and/or antagonists in the compound are peptides. In some embodiments, the at least one GPCR agonist and/or antagonist peptide has an N-terminal acetyl group, a C-terminal carboxamide group, or the combination thereof.

Thus, in one aspect, the invention provides a compound comprising at least one G-protein coupled receptor (GPCR) agonist and/or antagonist covalently bound to at least one oral bioavailability enhancing moiety and/or protein binding enhancing moiety. In some embodiments, the at least one GPCR agonist and/or antagonist is a GLP-1 agonist or antagonist, a GIP agonist or antagonist, or a CRLR agonist or antagonist. In some embodiments, the at least one GPCR agonist is a GLP-1 agonist covalently bound to a GIP agonist. In some embodiments, the at least one GPCR agonist and/or antagonist is a peptide.

In some embodiments, the at least one oral bioavailability enhancing moiety and/or protein binding enhancing moiety is independently bound to an N-terminal amino acid residue, to a C-terminal amino acid residue, or to any non-terminal amino acid residue of the at least one GPCR agonist and/or antagonist, or introduced or replaced therein, or bound to any other component thereof. In some embodiments, the non-terminal amino acid residue is a lysine.

In some embodiments, the at least one oral bioavailability enhancing moiety and/or protein binding enhancing moiety comprises a bile salt or bile acid. In some embodiments, the bile salt or bile acid comprises lithocholic acid, cholic acid, chenodeoxycholic acid, deoxycholic acid, ursodeoxycholic acid, or 7-ketolithocholic acid. In some embodiments, a plurality of bile acids or bile salts are covalently bound to the GPCR agonist and/or antagonist.

In some embodiments, the oral bioavailability enhancing moiety and/or protein binding enhancing moiety is (B) or (Z): 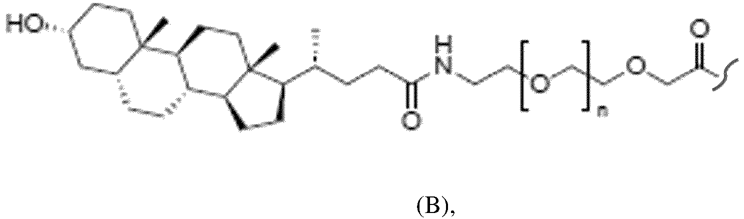 wherein n is between 0 and about 100; 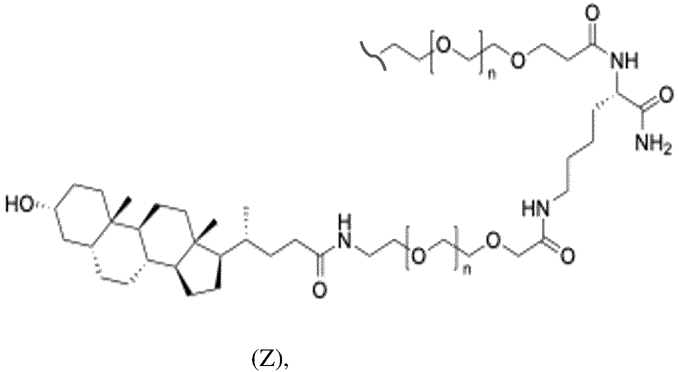 wherein each n is independently between 0 and about 100.

In some embodiments, the at least one GPCR agonist is GLP-1, exenatide, liraglutide, albiglutide, dulaglutide, semaglutide, lixisenatide, efpeglenatide or tirzepatide.

In some embodiments, the at least one GPCR agonist and/or antagonist has an N-terminal acetyl group, a C-terminal carboxamide group, or the combination thereof.

In some embodiments, the at least one GPCR agonist is semaglutide, and the at least one oral bioavailability enhancing moiety and/or protein binding enhancing moiety comprises lithocholic acid. In some embodiments, the oral bioavailability enhancing moiety and/or protein binding enhancing moiety is (B) or (Z): 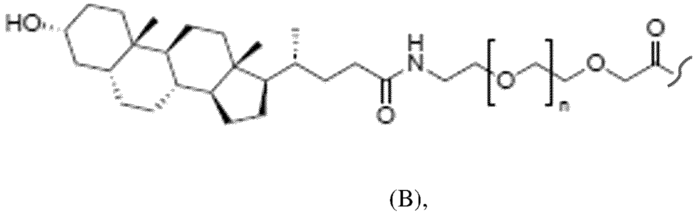 wherein n is between 0 and about 100; 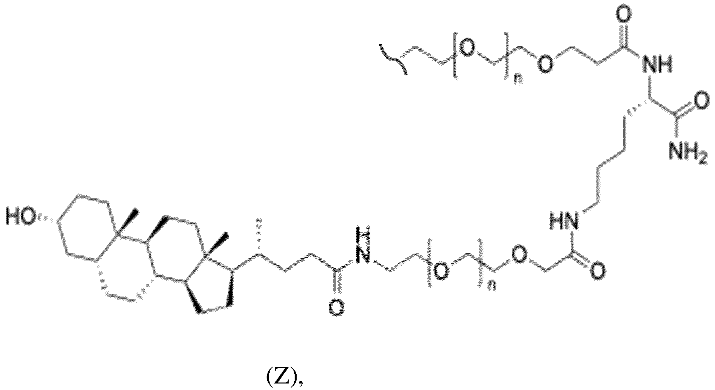 wherein each n is independently between 0 and about 100.

In some embodiments, the moiety comprising the lithocholic acid is bound to the C terminus of semaglutide. In some embodiments, the compound is Compound (I-A) 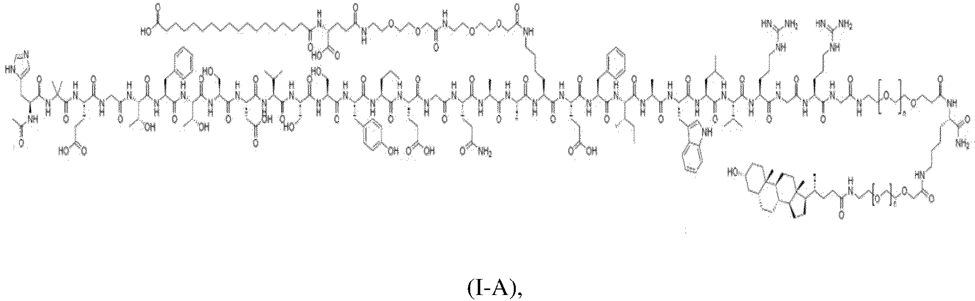 wherein each n is independently between 0 and about 100.

In some embodiments, each n is independently about 10 to about 20. In some embodiments of Compound I-A, each occurrence of n is about 20. In some embodiments of Compound I-A, each occurrence of n is about 10. In some embodiments of Compound I-A, each occurrence of n is 20. In some embodiments of Compound I-A, each occurrence of n is 10. In some embodiment, each occurrence of n is independently 2, 4, 8 or 12.

In some embodiments, the moiety comprising lithocholic acid is bound to a non-terminal amino acid of semaglutide. In some embodiments, the amino acid is lysine. In some embodiments, the compound is Compound (I-B) 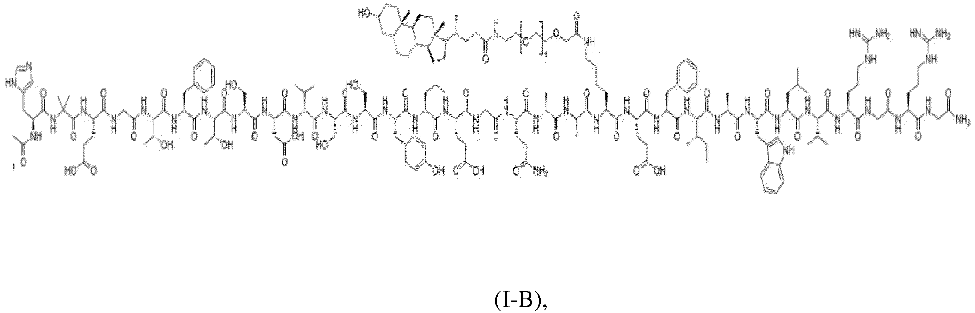 wherein n is from 0 to about 100.

In some embodiments, n is about 10 to about 20. In some embodiments of Compound I-B, n is about 10. In some embodiments of Compound I-B, n is about 20. In some embodiments of Compound I-B, n is 10. In some embodiments of Compound I-B, n is 20. In some embodiment, n is 2, 4, 8 or 12.

In some embodiments, the moiety comprising the lithocholic acid is bound to a non-terminal amino acid and to the C-terminal amino acid of semaglutide. In some embodiments, the compound is Compound (I-C) 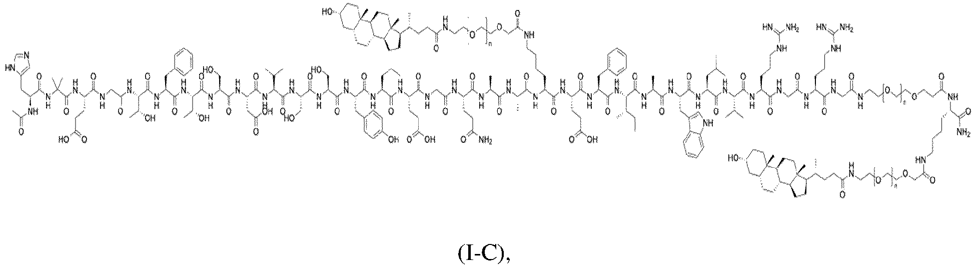 wherein each n is independently from 0 to 100.

In some embodiments, each n is independently about 10 to about 20. In some embodiments of Compound I-C, each n is about 20. In some embodiments of Compound I-C, each n is about 10. In some embodiment, each occurrence of n is independently 2, 4, 8 or 12.

In some embodiments, the at least one GPCR agonist is tirzepatide, and the at least one oral bioavailability enhancing moiety and/or protein binding enhancing moiety comprises lithocholic acid.

In some embodiments, the CRLR agonist is adrenomedullin, amylin or an analogue thereof.

In one aspect, a pharmaceutical composition is provided comprising any compound described herein and a pharmaceutically acceptable buffer, diluent, excipient, vehicle or carrier. In some embodiments, the pharmaceutical composition is formulated for oral administration.

In one aspect, a compound described herein or a pharmaceutical composition of any compound described herein is provided for use in treating a GPCR agonist and/or antagonist responsive condition or disease by administering the compound or the composition to a subject in need thereof. In some embodiments, the administering is oral. In some embodiments, the circulating half-life and/or the exposure of the compound is increased. In some embodiments, the GPCR agonist and/or antagonist responsive condition or disease is type 2 diabetes, obesity, insulin resistance, osteoporosis, migraine, Parkinson's disease, Alzheimer's disease, stroke, multiple sclerosis, traumatic brain injury, cardiovascular disease, hormone imbalance, reproductive endocrine axis dysfunction, or cancer.

Other features and advantages of the present invention will become apparent from the following detailed description examples and figures. It should be understood, however, that the detailed description and the specific examples while indicating preferred embodiments of the invention are given by way of illustration only, since various changes and modifications within the spirit and scope of the invention will become apparent to those skilled in the art from this detailed description.

### BRIEF DESCRIPTION OF THE FIGURE

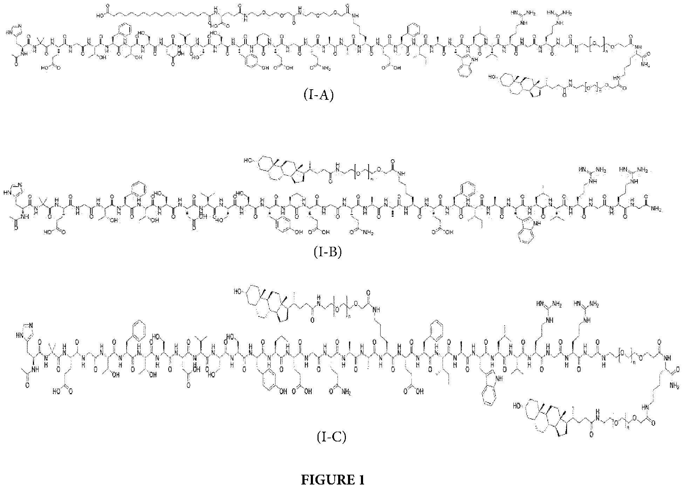

**FIGURE 1** depicts Compounds (I-A), (I-B) and (I-C).

### DETAILED DESCRIPTION OF THE INVENTION

The present subject matter may be understood more readily by reference to the following detailed description which forms a part of this disclosure. It is to be understood that this invention is not limited to the specific products, methods, conditions or parameters described and/or shown herein, and that the terminology used herein is for the purpose of describing particular embodiments by way of example only and is not intended to be limiting of the claimed invention.

Drugs targeting G-protein coupled receptors (GPCRs) have been developed to treat various diseases, including cancer, immunological diseases, cardiovascular disease, neurological disease, and metabolic diseases. Developing GPCR agonists or antagonists having a therapeutic potency for oral delivery remains a substantial challenge.

The disclosure is directed to compounds, and their pharmaceutical compositions and uses, comprising at least one GPCR receptor agonist or antagonist covalently bound to at least one moiety that enhances oral bioavailability of the compound and/or enhances protein binding of the compound. In one embodiment, enhanced oral bioavailability comprises increasing absorption after oral administration, or providing or increasing biological activity within the body after oral administration. In one embodiment, enhancing protein binding comprises providing or increasing binding of the orally absorbed compound to circulating plasma proteins such as but not limited to albumin. Such modifications of the GPCR agonist or antagonist in one embodiment increase and/or prolong circulating half-life, exposure, activity or efficacy. In one embodiment, enhancing protein binding comprises providing or increasing binding of the parenterally, e.g., subcutaneously administered compound to circulating plasma proteins such as but not limited to albumin. Such modifications of the GPCR agonist or antagonist in one embodiment increase and/or prolong circulating half-life, exposure, activity or efficacy.

Such compounds provide or increase the biological activity or activities of the GPCR receptor agonist or antagonist by oral administration of the compound, or by other routes of administration. Biological activities of GPCR receptor agonists and antagonists include but are not limited to various metabolic activities for treatment of various diseases and conditions, as well as improving normal biological activities and functions. For example, an agonist of the glucagon-like peptide 1 receptor (GLP-1R) stimulates insulin production. An agonist of the glucose-dependent insulinotropic peptide receptor (GIPR) also increases insulin production. Agonists of the calcitonin receptor-like receptor (CRLR) include but are not limited to adrenomedullin and amylin. Such GPCR agonists, and compounds disclosed embodying them, may be used to treat, by oral administration, conditions and diseases such as but not limited to type 2 diabetes, obesity, insulin resistance, osteoporosis, migraine, Parkinson's disease, Alzheimer's disease, stroke, multiple sclerosis, traumatic brain injury and cardiovascular disease. Antagonists of GPCR receptors are useful for blocking hormone activity, manipulation of the reproductive endocrine axis, and treatment of cancer.

Typically, GPCR agonists and antagonists and in particular peptide GPCR agonists and antagonists, require parenteral administration for activity. The present disclosure teaches modifications of GPCR agonists and antagonists, and in particular of peptide GPCR agonists and antagonists, which are orally bioavailable and can be administered orally to a subject to achieve the desired pharmacological activities. In some embodiments, administration of the compounds disclosed herein may be parenteral, with the advantage of increased bioavailability, circulating half-life, exposure, activity or efficacy, or any combination thereof.

### MOIETIES THAT ENHANCE ORAL BIOAVAILABILITY

In some embodiments, a moiety covalently bound to the at least one GPCR agonist or antagonist increases oral bioavailability of the GPCR agonist or antagonist. In some embodiments, a moiety covalently bound to the at least one peptide GPCR agonist or antagonist increases oral bioavailability of the peptide GPCR agonist or antagonist. In one embodiment, the moiety that increases oral bioavailability comprises a bile salt or bile acid. In some embodiments, the moieties that increase oral bioavailability also increase protein binding. In other embodiments, the moiety is a fatty acid or derivative thereof, a lipophilic compound, a medium chain fatty acid, a surfactant, or a calcium chelator.

In some embodiments, the oral bioavailability enhancing moiety is an enhancer of transcellular transport or an enhancer or paracellular transport. Non-limiting examples of enhancers of transcellular transport include a fatty acid or a derivative thereof, e.g., oleic acid or an acylcarnitine. Non-limiting examples of enhancers of transcellular transport include a lipophilic compound, e.g., a lipophilic agent enhancing membrane permeability. Non-limiting examples of enhancers of paracellular transport include a medium chain fatty acid, e.g., capric acid; or a surfactant e.g., sodium caprate or sodium lauryl sulfate; or a calcium chelator, e.g., ethylenediaminetetraacetic acid (EDTA) or 1,4,7,10-tetraazacyclododecane-1,4,7,10-tetraacetic acid (DOTA); or a bile salt such as those described herein, that modulate tight junctions.

### Bile acids and Bile Salts

In one embodiment, at least one bile acid or bile salt is conjugated to a GPCR agonist or antagonist as disclosed herein. In one embodiment, at least one bile acid or bile salt is conjugated to a peptide GPCR agonist or antagonist as disclosed herein. Non-limiting examples of bile acids useful for such purposes include but are not limited to cholic acid, lithocholic acid, deoxycholic acid, 7-ketolithocholic acid, ursodeoxycholic acid, chenodeoxycholic acid, deoxycholic acid, ursodeoxycholic acid, taurocholic acid, glycocholic acid, tauro-chenodeoxycholic acid, glyco-chenodeoxycholic acid, tauro-deoxycholic acid, glyco-deoxycholic acid, tauro-ursodeoxycholic acid, and glyco-ursodeoxycholic acid. Non-limiting examples of keto derivatives of bile acids also useful for such purposes include but are not limited to 12-monoketocholic acid, 7-monoketocholic acid, 7,12-diketocholic acid, 3,7,12-triketocholic acid, and 12-monoketodeoxycholic acid.

Such bile acid or bile salt may comprise a moiety that is bound to the GPCR agonist or antagonist, such that the bile acid or salt is part of the moiety pendant to the GPCR agonist or antagonist. As described herein, in some embodiments, the moiety comprising the bile acid or bile salt also comprises one or more spacers, such as PEG, between the bile acid or bile salt moiety and the GPCR agonist or antagonist. While such conjugated bile acid or bile salt may be referred to as a bile acid or bile salt, or as comprising a bile acid or bile salt, the disclosure is not so limited as to the structure of the moiety comprising the bile acid or bile salt, such that the moiety imparts the enhancement of oral bioavailability and/or enhancement of protein binding.

Any one or more bile acid may be covalently conjugated to the one or more GPCR agonist or antagonist of the compound via a reactive group on the bile acid, such as a carboxylic acid or hydroxyl group. By way of non-limiting example, lithocholic acid comprises a 3α hydroxyl group and a carboxylic acid group available for conjugation as disclosed herein. As will be described herein, such conjugation to the GPCR agonist or antagonist may be through a reactive moiety on the GPCR agonist or antagonist, such as a side chain of an amino acid thereof.

### Fatty Acids and Derivatives

Any one or more fatty acids or derivatives may be covalently conjugated to the one or more GPCR agonist of the compound. Fatty acids may be saturated, monounsaturated or polyunsaturated. Non-limiting examples of fatty acids include: caproic acid; heptanoic acid, caprylic acid, capric acid, 10-undecenoic acid, lauric acid, 5-dodecenoic acid, myristic acid, myristoleic acid, palmitic acid, palmitoleic acid, margaric acid, 10-heptadecenoic acid, stearic acid, nonadecanoic acid, 10-nonadecenoic acid, arachidic acid, eicosanoic acid, erucic acid, and oleic/vaccenoic acid. Derivatives of fatty acids include, among others, oxylipins, hydroxy fatty acids, diols, alkenones and wax esters. Others are described in Resh et al., 2013, Covalent lipid modification of proteins, Curr Biol 23(10):R431-R435, incorporated herein by reference.

### Lipophilic Compounds

Any one or more lipophilic compounds may be covalently conjugated to the one or more GPCR agonist or antagonist of the compound as described herein. Non-limiting examples of lipophilic compounds include those described in Angelova et al., 1999, Journal of Biotechnology 67:13-32, incorporated herein by reference.

### Medium Chain Fatty Acids

Any one or more medium chain fatty acids may be covalently conjugated to the one or more GPCR agonist or antagonists of the compound. Non-limiting examples include caproic acid, heptanoic acid, caprylic acid, capric acid, and 10-undecenoic acid.

### Surfactants

Any one or more surfactants may be covalently conjugated to the one or more GPCR agonist or antagonist of the compound.

### Calcium Chelators

Any one or more calcium chelators may be covalently conjugated to the one or more GPCR agonist or antagonist of the compound, such as but not limited to EDTA (ethylenediaminetetraacetic acid) and DOTA (tetraxetan; 1,4,7,10-tetraazacyclododecane-1,4,7,10-tetraacetic acid).

### COVALENT CONJUGATION

Various methods may be used to covalently conjugate at least one oral bioavailability enhancing moiety or protein binding enhancing moiety to at least one GPCR agonist or antagonist as disclosed herein. Such covalent conjugation may comprise activation of a reactive group on one component and direct covalent binding to the other, such as through a peptide bond, or in other embodiments, a homobifunctional or heterobifunctional cross-linker may be used that links the two components. In some embodiments, a longer linker is utilized, such as polyethylene glycol. Any such methods and resulting compounds are fully embraced by the present disclosure. As used herein linker generally refers to the moiety used to bind the one or more oral bioavailability enhancing moiety or protein binding enhancing moiety to the GPCR agonist or antagonist. Spacer, as used herein, generally refers to polymers or other linear moieties that extend the length of the oral bioavailability enhancing moiety or protein binding enhancing moiety, within the moiety.

In some embodiments, an epsilon amino group of a lysine in the GPCR agonist or antagonist peptide sequence may be used for conjugation to the oral bioavailability enhancing or protein binding enhancing moiety. In non-limiting embodiments, a lysine at position 20 in the following peptides may be a site for conjugation: GLP-1, liraglutide, albiglutide, dulaglutide, semaglutide and tirzepatide. Other amino acids on these or other peptides can be used for conjugation provided conjugation using that amino acid does not interfere with the biological activity of the peptide.

Cross-linking agents such as carbodiimides may be used to covalently bind carboxyl and amino groups to form a peptide bond. Examples of carbodiimides are 1-cyclohexyl-3-(2-morpholinyl-(4-ethyl) carbodiimide (CMC), 1-ethyl-3-(3-dimethyaminopropyl) carbodiimide (EDC) and 1-ethyl-3-(4-azonia-44-dimethylpentyl) carbodiimide. Other means of direct conjugation are fully embraced herein.

In other embodiments, covalent cross-linking of the GPCR agonist or antagonist to the oral bioavailability enhancing moiety or protein binding enhancing moiety comprises using a reagent that introduces at least one atom therebetween. As noted herein, any site on each of the foregoing one or more of either component may be covalently bound to the other. In one non-limiting example, the cross-linking agent is or uses cyanogen bromide, glutaraldehyde and succinic anhydride. In other embodiments, any of a number of homo-bifunctional agents including a homo-bifunctional aldehyde, a homo-bifunctional epoxide, a homo-bifunctional imido-ester, a homo-bifunctional N-hydroxysuccinimide ester, a homo-bifunctional maleimide, a homo-bifunctional alkyl halide, a homo-bifunctional pyridyl disulfide, a homo-bifunctional aryl halide, a homo-bifunctional hydrazide, a homo-bifunctional diazonium derivative and a homo-bifunctional photoreactive compound can be used.

In other embodiments, hetero-bifunctional compounds, for example, compounds having an amine-reactive and a sulfhydryl-reactive group, compounds with an amine-reactive and a photoreactive group and compounds with a carbonyl-reactive and a sulfhydryl-reactive group.

In other embodiments, the homo-bifunctional cross-linking agents include the bifunctional N-hydroxysuccinimide esters dithiobis(succinimidylpropionate), disuccinimidyl suberate, and disuccinimidyl tartarate; the bifunctional imido-esters dimethyl adipimidate, dimethyl pimelimidate, and dimethyl suberimidate; the bifunctional sulfhydryl-reactive crosslinkers 1,4-di-[3'-(2'-pyridyldithio)propionamido]butane, bismaleimidohexane, and bis-N-maleimido-1, 8-octane; the bifunctional aryl halides 1,5-difluoro-2,4-dinitrobenzene and 4,4'-difluoro-3,3'-dinitrophenylsulfone; bifunctional photoreactive agents such as bis-[b-(4-azidosalicylamido)ethyl] di sulfide; the bifunctional aldehydes formaldehyde, malondialdehyde, succinaldehyde, glutaraldehyde, and adipaldehyde; a bifunctional epoxide such as 1,4-butaneodiol diglycidyl ether; the bifunctional hydrazides adipic acid dihydrazide, carbohydrazide, and succinic acid dihydrazide; the bifunctional diazoniums o-tolidine, diazotized and bis-diazotized benzidine; the bifunctional alkylhalides N1N'-ethylene-bis(iodoacetamide), N1N'-hexamethylene-bis(iodoacetamide), N1N'-undecamethylene-bis(iodoacetamide), as well as benzylhalides and halomustards, such as ala'-diiodo-p-xylene sulfonic acid and tri(2-chloroethyl)amine, respectively.

In other embodiments, hetero-bifunctional cross-linking agents used to link the components, as described herein, include, but are not limited to, SMCC (succinimidyl-4-(N-rnaleimidomethyl)cyclohexane-1-carboxylate), MBS (m-maleimidobenzoyl-N-hydroxysuccinimide ester), SIAB (N-succinimidyl(4-iodoacteyl)aminobenzoate), SMPB (succinimidyl-4-(p-maleimidophenyl)butyrate), GMBS (N-(.gamma.-maleimidobutyryloxy)succinimide ester), MPBH (4-(4-N-maleimidopohenyl) butyric acid hydrazide), M2C2H (4-(N-maleimidomethyl) cyclohexane-1-carboxyl-hydrazide), SMPT (succinimidyloxycarbonyl-a-methyl-a-(2-pyridyldithio)toluene), and SPDP (N-succinimidyl 3-(2-pyridyldithio)propionate).

### SPACERS

As noted herein, the term spacer generally refers to a polymer or other linear moiety that increases the distance that the oral bioavailability enhancing moiety or protein binding enhancing moiety is located from its site of attachment to the GPCR agonist or antagonist. Where there is more than one spacer in a compound, each may independently comprise a different moiety or be of a polymer of a different length. For example, the length of the poly(ethylene glycol) in moiety (B) may be between 0 and 100 ethylene glycol monomers. In another example, in moiety (Z), each poly(ethylene glycol) spacer may be independently between 0 and about 100 ethylene glycol monomers. When two same or different oral bioavailability enhancing moiety or protein binding enhancing moieties are on the same GPCR agonist or antagonist, each spacer length may be independently between 0 and about 100. In some embodiments, each n is between 0 and about 10. In some embodiments, each n is between 0 and about 20. In some embodiments, each n is between 0 and 10. In some embodiments, each n is between 0 and 20. In some embodiments, each n is independently 2, 4, 8 or 12. In some embodiments, each n is independently 0, 1, 2, 3, 4, 5, 6, 7, 8, 9, 10, 11, 12, 13, 14, 15, 16, 17, 18, 19, or 20.

For example, in the oral bioavailability enhancing moiety or protein binding enhancing moieties (B) and (Z) below, 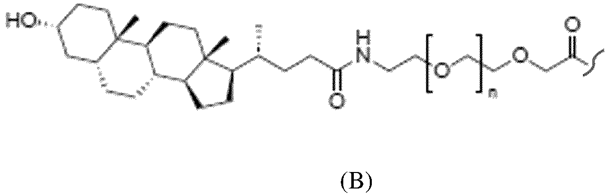 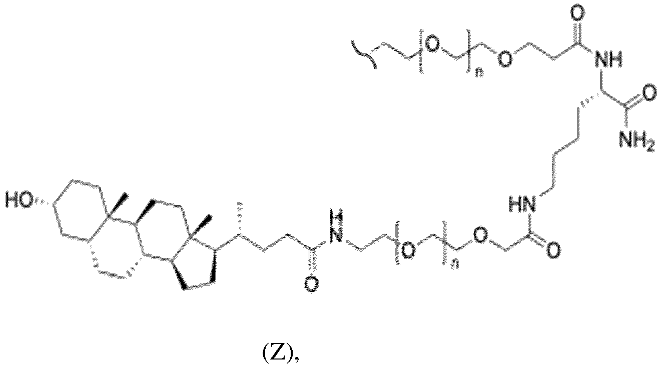 each n may be independently between 0 and about 100. In some embodiments, each n is between 0 and about 10. In some embodiments, each n is between 0 and about 20. In some embodiments, each n is between 0 and 10. In some embodiments, each n is between 0 and 20. In some embodiments, each n is independently 2, 4, 8 or 12. In some embodiments, each n is independently 0, 1, 2, 3, 4, 5, 6, 7, 8, 9, 10, 11, 12, 13, 14, 15, 16, 17, 18, 19, or 20. If a GPCR agonist or antagonist has both a (B) and (Z) moiety, the one or more n on each moiety may independently be any of the foregoing values, independent of the n on the other moiety.

As noted herein, for the spacer being, in one non-limiting embodiment polyethylene glycol (also referred to as poly(ethylene glycol)), each such spacer when more than one is present in a compound, may each have a different length, for example, from no ethylene glycol to about 100. In some embodiments, no spacer is present at one or more locations.

In one embodiment, the one or more oral bioavailability enhancing moiety or protein binding enhancing moiety is covalently bound to the one or more GPCR agonist or antagonist through a linker, such that the oral bioavailability enhancing moiety or protein binding enhancing moiety is pendant from the GPCR agonist or antagonist by an intervening linker, or two (or more) GPCR agonists and/or antagonists are linked through linkers.

As noted herein, the spacer may be a linear polymer such as ethylene glycol subunits between a bile acid steroid portion of an oral bioavailability enhancing moiety or protein binding enhancing moiety, and the carboxylic acid moiety for cross-linking (e.g., linking to the GPCR agonist or antagonist). In some embodiments, the polymer is 0 to about 100 monomers in length. In some embodiments, the polymer is 0 to about 100 atoms in length. In some embodiments, the spacer is about 2 monomers, about 4 monomers, about 5 monomers, about 6 monomers, about 8 monomers, about 10 monomers, about 20 monomers, about 30 monomers, about 40 monomers, about 50 monomers, about 60 monomers about 70 monomers, about 80 monomers, about 90 monomers, or about 2 atoms, about 4 atoms, about 5 atoms, about 6 atoms, about 8 atoms, 10 atoms, about 20 atoms, about 30 atoms, about 40 atoms, about 50 atoms, about 60 atoms about 70 atoms, about 80 atoms, about 90 atoms.

In some embodiments, the spacer or linker is a polyethylene glycol (PEG), an amino acid, a hydrocarbon with terminal carboxylic acid and amine groups, or a combination thereof. In some embodiments, the amino acid is a canonical or nonstandard amino acid. In some embodiments, the hydrocarbon with terminal carboxylic acid and amine groups is 5-amino pentanoic acid, 10-amino decanoic acid, or 18-amino octadecanoic acid. Other spacers may comprise amino acids or amino acid polymers.

In some embodiments, the spacer is a polyethylene glycol (PEG). In some embodiments, the spacer is PEG2, PEG4, PEG8, or PEG12, having 2, 4, 8 or 12 ethylene glycol units. In other embodiments, the spacer is 2xGGGGS (GGGGSGGGGS, SEQ ID NO:1), 4xGGGGS (GGGGSGGGGSGGGGSGGGGS, SEQ ID NO:2), or 8xGGGGS (GGGGSGGGGSGGGGSGGGGSGGGGSGGGGSGGGGSGGGGS, SEQ ID NO:3).

In some embodiments, the compound of the invention further conjugates a fatty diacid, e.g., a C16 diacid, to a residue on the peptide of the compound to increase plasma half-life.

The disclosure is not so limited as to the means for covalently crosslinking the one or more GPCR agonists or antagonists to the one or more oral bioavailability enhancing moiety or protein binding enhancing moiety, or linking two (or more) GPCR agonists to themselves, or linking two (or more) oral bioavailability enhancing moiety or protein binding enhancing moiety to themselves, and one of skill in the art will be cognizant of ways to accomplish the cross-linking. The disclosure is further not so limited as to the number of either type of component or the arrangement of where the components are joined. The inclusion of a linker as described herein is not necessarily a component and in some embodiments, the oral bioavailability enhancing moiety or protein binding enhancing moiety may be conjugated directly to the peptide.

### MOIETIES THAT ENHANCE PROTEIN BINDING

In one embodiment, a moiety that enhances protein binding of the GPCR agonist or antagonist comprises increasing the binding thereof to serum and/or plasma proteins in a human or other mammal, such as human serum albumin. Enhancing protein binding in one embodiment increases circulating half-life, bioavailability, biological activity, by way of non-limiting examples. Such properties may be imparted to the GPCR agonist or antagonist regardless of the route of administration. In some embodiments, a moiety that enhances oral bioavailability may also enhance protein binding.

In one embodiment, the moiety that enhances protein binding is at least one bile acid or bile salt, as described herein, and covalently bound to the at least one GPCR agonist as described herein.

In other embodiments, the moiety comprises any of the herein described moieties that enhances bioavailability, such as but not limited to a bile acid, a fatty acid, e.g., a C16 diacid, or fatty acid derivative, a lipophilic compound, a medium chain fatty acid, a surfactant, or a calcium chelator.

### GPCR AGONISTS

G protein-coupled receptors (GPCRs) are located on the cell membrane where they transduce extracellular signals into key physiological effects. Their endogenous ligands include odors, hormones, neurotransmitters, chemokines, etc., varying from photons, amines, carbohydrates, lipids, peptides to proteins. GPCRs have been implicated in a large number of diseases, such as but not limited to type 2 diabetes mellitus (T2D or T2DM), obesity, depression, cancer, and Alzheimer's disease, as described by Yan et al., Signal Transduction and Targeted Therapy, 2021; 6:7, which is incorporated herein by reference in its entirety.

In some embodiments, a GPCR agonist disclosed herein is a peptide. In any of the GPCR agonist peptides described herein, the disclosure extends to analogues of such peptides that may have variations in one of more amino acids, an N-terminal acetylation, a C-terminal amidation, or any combination thereof, such modifications provided to, in some embodiments, enhance the biological activity of the agonist. The description herein of any GPCR agonist peptide sequence is not intended to be limited to such sequence but embraces amino acid variations, mutations, deletions, additions, pendant side chains, conjugation with e.g., a fatty acid, etc.

### GLP-1 receptor agonists

Non-limiting examples include GLP-1(7-37), exenatide, liraglutide, albiglutide, dulaglutide, semaglutide, lixisenatide, efpeglenatide and tirzepatide. Their amino acid sequences are shown below (excluding any non-peptide or non-GPCR binding components). Any may exist as the C-terminal amide or free carboxylic acid.

| **Peptide** | **Sequence** |
|---|---|
| GLP-1(7-37) | HAEGTFTSDVSSYLEGQAAKEFIAWLVKGRG (SEQ ID NO:4) |
| Exenatide | HGEGTFTSDLSKQMEEEAVRLFIEWLKNGG PSSGAPPPS (SEQ ID NO:5) |
| Liraglutide | HAEGTFTSDVSSYLEGQAAKEFIAWLVKGRG (SEQ ID NO:6) |
| Albiglutide | HGEGTFTSDVSSYLEGQAAKEFIAWLVKGRG (SEQ ID NO:7) |
| Dulaglutide | HGEGTFTSDVSSYLEEQAAKEFIAWLVKGGG (SEQ ID NO:8) |
| Semaglutide | HXEGTFTSDVSSYLEGQAAKEFIAWLVRGRG (SEQ ID NO:9) wherein X represents α-aminoisobutyric acid (AIB) |
| Lixisenatide | HGEGTFTSDLSKQMEEEAVRLFIEWLKNGGPSSGAPPSKK KKKK (SEQ ID NO:10) |
| Efpeglenatide | GEGTFTSDLSKQMEEEAVRLFIEWLKNGGPSSGAPPPS (SEQ ID NO:11) |
| Tirzepatide | YXEGTFTSDYSIXLDKIAQKAFVQWLIAGGPSSGAPPPS (SEQ ID NO:12) wherein each X represents α-aminoisobutyric acid (AIB) |

Other examples include GLP-1(7-36)NH2 (HAEGTFTSDVSSYLEGOAAK EFIAWLCKGR-NH2, SEQ ID NO:13), GLP-1(1-36) (HDEFERHAEGTFTSDVSSYLEGQAAKEFIAWLVKGR, SEQ ID NO:14), Exendin-4 (HGEGTFTSDLSKQMEEEAVRLFIEWLKNGGPSSGAPPPS, SEQ ID NO:15), GLP-1(7-37) (HAEGTFTSDVSSYLEGQAAKEFIAWLVKGRG, SEQ ID NO:16), GLP-1(Gly8) (HDEFERHGEGTFTSDVSSYLEGQAAKEFIAWLVKGR, SEQ ID NO:17), GLP-1(Ala8) (HDEFERHAEGTFTSDVSSYLEGQAAKEFIAWLVKGR, SEQ ID NO:53), GLP-1 (AIB8) (HDEFERHXEGTFTSDVSSYLEGQAAKEFIAWLVKGR, wherein X is α-aminoisobutyric acid, SEQ ID NO:54), GLP-1(1-36)NH2 (HDEFERHAEGTFTSDVSSYLEGQAAKEFIAWLVKGR-NH2, SEQ ID NO:18), and GLP-1(9-36)NH2 (EGTFTSDVSSYLEGQAAKEFIAWLVKGR-NH2, SEQ ID NO:19). Such GLP-1 agonists may have one or more variations in the amino acid sequence or modified amino acids but retain GLP-1 agonist activity. In one embodiment, semaglutide comprises at its second amino acid position an aminoisobutyric acid or a D-alanine. Any may exist as the C-terminal amide or free carboxylic acid.

In one embodiment, the GLP-1 agonist is GLP-1.

In some embodiments, the GLP-1 agonist is also a GIPR agonist.

### GIPR agonists

Non-limiting examples include GIP(1-42) (YAEGTFISDYSIAMDKIHQQDFVNWLLAQKGKKNDWKHNITQ, SEQ ID NO:20), GIP(1-30)NH2 (YAEGTFISDYSIAMDKIHQQDFVNWLLAQKG-NH2, SEQ ID NO:21), GIP(2-30)NH2 (AEGTFISDYSIAMDKIHQQDFVNWLLAQKG-NH2, SEQ ID NO:22), Pro3(GIP) (YAPGTFISDYSIAMDKIRQQDFVNWLLAQRGKKSDWKHNITQ, SEQ ID NO:23)), GIP(3-30)NH2 (EGTFISDYSIAMDKIHQQDFVNWLLAQKG-NH2, SEQ ID NO:24), GIP(3-42) (EGTFISDYSIAMDKIHQQDFVNWLLAQKGKKNDWKHNITQ, SEQ ID NO25), CY-5 (HXQGTFTSDYSKXLDKRAAKDFVEWLKNGGPSSGAPPPS-NH2, X is AIB; SEQ ID NO:26), Peptide 19 (YKLDKFYDFYGLISSSYPA; SEQ ID NO:52), DA-JC4 (YXEGTFTSDYSIYLDKQAAXEFVNWLLAGGPSSGAPPPSKKKKKK-NH2, X is AIB; SEQ ID NO:27), and tirzepatide. Such GIP agonists may have one or more variations in the amino acid sequence or modified amino acids but retain GIP agonist activity. In one embodiment, semaglutide comprises at its second amino acid position an aminoisobutyric acid or a D-alanine. Any may exist as the C-terminal amide or free carboxylic acid.

### Dual GLP-1 and GIPR agonists

Non-limiting examples include tirzepatide, semaglutide, liraglutide, dulaglutide, exenatide.

### CRLR agonists

Non-limiting CRLR agonists include amylin and adrenomedullin.

### GPCR ANTAGONISTS

Non-limiting examples of GPCR antagonists that may be modified as described herein include but are not limited to those described in Davenport et al., 2020, Advances in therapeutic peptides targeting G protein-coupled receptors, Nature Reviews Drug Discovery 19:389-413. Examples useful for the purposes disclosed herein include degarelix, abarelix, buserelin, icatibant, cetrorelix, goserelin, gonadorelin, histrelin, nafarelin, triptorelin, leuprolide, ganirelix, BL-8040, plerixafor, barusiban, ozarelix and nemifitide.

Any of the foregoing GPCR agonists or antagonists and in particular peptide agonists or antagonists may be an analogue or variant thereof, or may be modified (independently from the modifications described elsewhere herein). In some embodiments, the modifications include wherein the peptide comprises at least one modified amino acid, wherein the modification is any of acetylation, amidation, C-linked glycosylation, citrullination, crotonylation, formylation, glutarylation, glutathionylation, hydroxylation, lipidation, malonylation, methylation, myristoylation, N-linked glycosylation, O-linked glycosylation, oxidation, palmitoylation, phosphorylation, pyrrolidone carboxylic acid, S-nitrosylation, sulfation, or sumoylation. In some embodiments, the polypeptide units is N-terminus acetylated. In some embodiments, the polypeptide comprises one or more non-canonical amino acid, such as 2-aminoisobutyric acid. In another embodiment, any of the polypeptide units comprises one or more lipidated lysine residues.

In some embodiments, the sequence homology to the native version of the GPCR agonist or antagonist peptide is greater than about 75%, 80%, 85%, 90%, 95% or 99%.

### EXEMPLARY COMPOUNDS

In one embodiment, the GPCR agonist peptide semaglutide is covalently conjugated to a moiety comprising the oral bioavailability enhancer lithocholic acid. In one embodiment, the moiety comprising the lithocholic acid is bound via a poly(ethylene glycol) spacer. In one embodiment, the moiety comprising the lithocholic acid is bound to the C-terminus. In one embodiment, the following compound is provided: 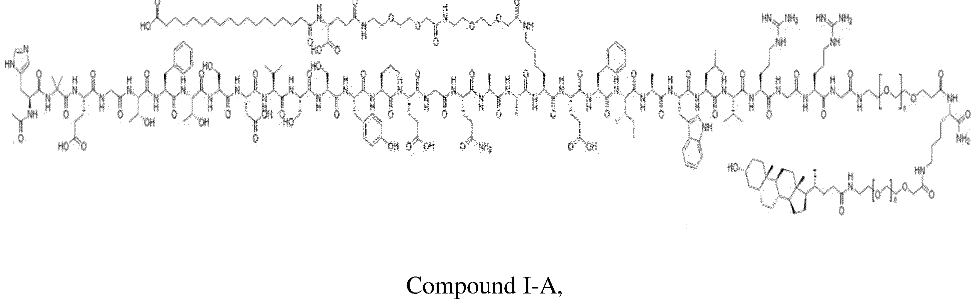 wherein each n is independently from 0 to about 100.

In one embodiment, each n is about 10. In one embodiment, each n is about 20. In one embodiment, each n is 10. In one embodiment, each n is 20. In some embodiments, each n is independently, or both are, 2, 4, 8 or 12.

In one embodiment, the moiety comprising the lithocholic acid is bound via a poly(ethylene glycol) spacer, and the moiety comprising the lithocholic acid is bound to an epsilon-amino group on a lysine of the GPCR agonist. In one embodiment, the GPCR agonist is semaglutide. In one embodiment, the following compound is provided: 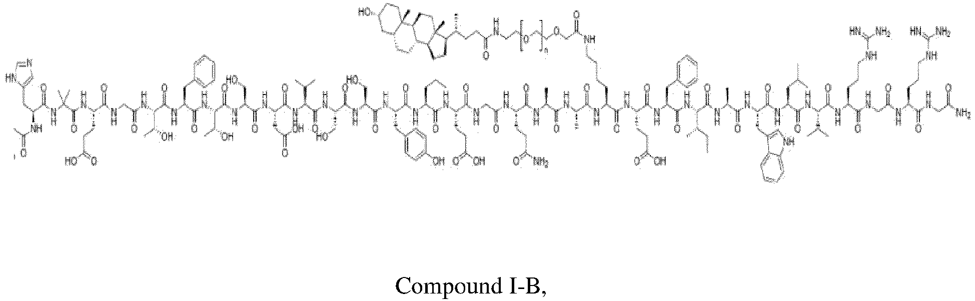 wherein n is from wherein n is from 0 to about 100.

In one embodiment, n is about 10. In one embodiment, n is about 20. In some embodiments, n is 2, 4, 8 or 12.

Such Compound I-C comprises semaglutide wherein the N at the C6 position of the lysine at position 20 has its [N-(17-carboxy-1-oxoheptadecyl)-L-γ-glutamyl-2-[2-(2-aminoethoxy)ethoxy]acetyl-2-[2-(2-aminoethoxy)ethoxy]acetyl] group substituted with moiety (B).

In one embodiment, a lithocholic acid is bound via a poly(ethylene glycol) spacer, and the lithocholic acid spacer is bound to an epsilon-amino group on a lysine of the GPCR agonist; and a lithocholic acid is bound via a poly(ethylene glycol) spacer, and the lithocholic acid spacer is bound to the C-terminus of the GPCR agonist. The following compound is provided: 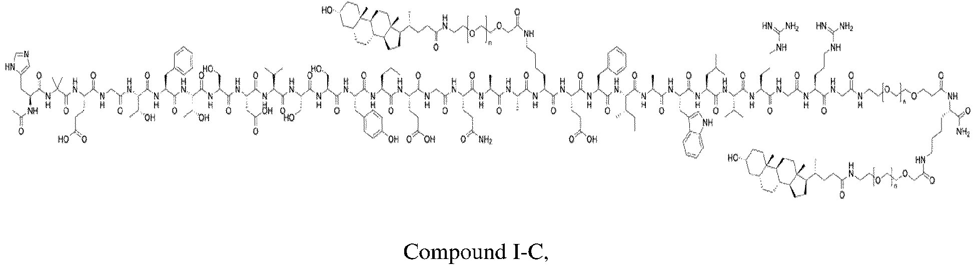 wherein each n is independently from 0 to about 100.

In one embodiment, each n is about 10. In one embodiment, each n is about 20. In one embodiment, each n is 10. In one embodiment, each n is 20. In some embodiments, each n is independently, or both are, 2, 4, 8 or 12 ethylene glycol units.

Such Compound I-C comprises semaglutide wherein the N at the C6 position of the lysine at position 20 has its [N-(17-carboxy-1-oxoheptadecyl)-L-γ-glutamyl-2-[2-(2-aminoethoxy)ethoxy]acetyl-2-[2-(2-aminoethoxy)ethoxy]acetyl] group substituted with moiety (B).

Similarly, any GPCR agonist or antagonist peptide or analogue thereof may have an oral bioavailability enhancing moiety or protein binding enhancing moiety such as a bile acid, for example a moiety comprising lithocholic acid, conjugated to the lysine at position 20 if present, or at another lysine, such as the moiety depicted as moiety (B): 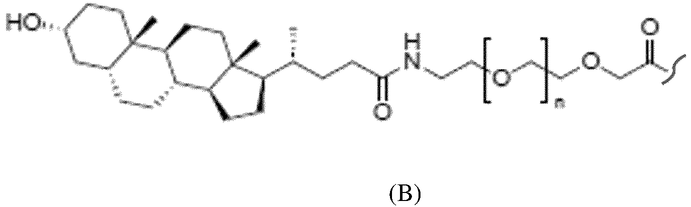 wherein n is from 0 to about 100.

In one embodiment, n is about 10. In one embodiment, n is about 20. In one embodiment, n is 10. In one embodiment, n is 20. In some embodiments, n is 2, 4, 8 or 12. In some embodiments, n is 0, 1, 2, 3, 4, 5, 6, 7, 8, 9, 10, 11, 12, 13, 14, 15, 16, 17, 18, 19, or 20.

Such GPCR agonist or antagonist peptide having an oral bioavailability enhancing moiety or protein binding enhancing (B) moiety may also have a C-terminal oral bioavailability enhancing moiety or protein binding enhancing moiety.

In some embodiments, any GPCR agonist or antagonist peptide or analogue thereof may have an oral bioavailability enhancing moiety or protein binding enhancing moiety bile acid such as a moiety comprising lithocholic acid conjugated to the C terminus. Such C-terminal oral bioavailability enhancing moiety or protein binding enhancing moiety, e.g., comprising a bile acid such as lithocholic acid, may include a spacer such as poly(ethylene glycol) as in the examples herein, or 2X, 4X or 8X GGGGS (SEQ ID NOS: 1, 2, and 3, respectively), or any combination of types of spacers. The C-terminal located oral bioavailability enhancing moiety or protein binding enhancing moiety acids may include a spacer such as poly(ethylene glycol) as in the examples above, or 2X, 4X or 8X GGGGS (SEQ ID NOS: 1, 2, and 3, respectively). For example, a C-terminal oral bioavailability enhancing moiety or protein binding enhancing moiety may comprise the following (Z) moiety comprising a lithocholic acid with a poly(ethylene glycol) spacer, and a poly(ethylene glycol) spacer moiety bound to the C-terminus of the peptide. 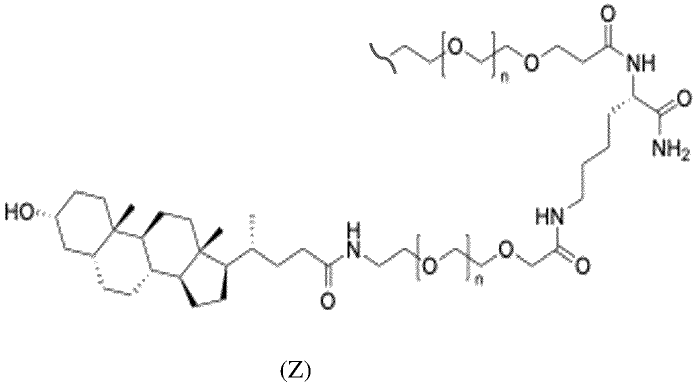 wherein each occurrence of n is independently selected from between 0 and about 100.

In some embodiments, each occurrence of n is independently selected from between 0 and about 100. In some embodiments, each occurrence of n is independently selected from between 0 and about 20. In some embodiments, each occurrence of n is independently selected from between about 10 and about 20. In some embodiments, each occurrence of n is independently selected from between 10 and 20. In some embodiments, each occurrence of n is 10. In some embodiments, each occurrence of n is 20. In some embodiments, each n is independently, or both are, 2, 4, 8 or 12 ethylene glycol units. In some embodiments, each n is independently 0, 1, 2, 3, 4, 5, 6, 7, 8, 9, 10, 11, 12, 13, 14, 15, 16, 17, 18, 19, or 20.

Such GPCR agonist or antagonist peptide having a C-terminal oral bioavailability enhancing moiety or protein binding enhancing moiety such as (Z), may also have an oral bioavailability enhancing moiety or protein binding enhancing moiety bound to a non C-terminal amino acid, such as to a lysine. Non-limiting examples include a (B) moiety. Thus, a GPCR agonist or antagonist peptide may have one or more (B) moieties, a (Z) moiety, or any combination thereof.

Such GPCR agonist or antagonist peptide having an oral bioavailability enhancing moiety or protein binding enhancing moiety bound to a non C-terminal amino acid, such as to a lysine, may also have a C-terminal oral bioavailability enhancing moiety or protein binding enhancing moiety. Non-limiting examples include a (B) moiety bound to a non-termina amino acid, and a (Z) moiety bound to the C terminus.

Thus, a GPCR agonist or antagonist peptide having an oral bioavailability enhancing moiety or protein binding enhancing moiety may have a C-terminal oral bioavailability enhancing moiety or protein binding enhancing moiety, at least one non-C-terminal oral bioavailability enhancing moiety or protein binding enhancing moiety, or both. In some embodiments, a GPCR agonist or antagonist peptide may have one or more (B) moieties, a (Z) moiety, or any combination thereof.

Non-limiting examples of GPCR agonists and antagonists comprising a non-C-terminal oral bioavailability enhancing moiety or protein binding enhancing moiety, examples comprising a C-terminal oral bioavailability enhancing moiety or protein binding enhancing moiety, or examples having both a non-C-terminal and a C-terminal oral bioavailability enhancing moiety or protein binding enhancing moiety are exemplified below. Lysine (K) followed by (B) indicates the (B) moiety is pendant from the lysine. A (Z) at the C terminus indicates the (Z) moiety is pendant from the C terminus. Other examples include such (B), (Z) or both present on any other GPCR agonist or antagonist peptide disclosed herein. In other embodiments, a GPCR agonist or agonist peptide may comprise a lipid such as [N-(17-carboxy-1-oxoheptadecyl)-L-γ-glutamyl-2-[2-(2-aminoethoxy)ethoxy]acetyl-2-[2-(2-aminoethoxy)ethoxy]acetyl] on the C6 position of a lysyl group, such as is shown pendant from lysine 20 on Compound I-B and Compound I-C. Such same or different lipid may be present on any GPCR agonist or antagonist disclosed herein provided at least one oral bioavailability enhancing moiety or protein binding enhancing moiety is also present. In some embodiments, such lipid is replaced by the oral bioavailability enhancing moiety or protein binding enhancing moiety as disclosed herein, such as in Compounds I-B and I-C.

| | |
|---|---|
| GLP-1(7-37) | HAEGTFTSDVSSYLEGOAAK(B)EFIAWLCKGRG (SEQ ID NO:28) |
| | HAEGTFTSDVSSYLEGOAAKEFIAWLCKGRG(Z) (SEQ ID NO:29) |
| | HAEGTFTSDVSSYLEGOAAK(B)EFIAWLCKGRG(Z) (SEQ ID NO:30) |
| Exenatide | HGEGTFTSDLSKQMEEEAVRLFIEWLK(B)NGGPSSGAPPPS(Z) (SEQ ID NO:31) |
| | HGEGTFTSDLSKQMEEEAVRLFIEWLKNGGPSSGAPPPS(Z) (SEQ ID NO:32) |
| | HGEGTFTSDLSKQMEEEAVRLFIEWLK(B)NGGPSSGAPPPS(Z) (SEQ ID NO:33) |
| Liraglutide | HAEGTFTSDVSSYLEGQAAK(B)EFIAWLVKGRG (SEQ ID NO:34) |
| | HAEGTFTSDVSSYLEGQAAKEFIAWLVKGRG(Z) (SEQ ID NO:35) |
| | HAEGTFTSDVSSYLEGQAAK(B)EFIAWLVKGRG(Z) (SEQ ID NO:36) |
| Albiglutide | HGEGTFTSDVSSYLEGQAAK(B)EFIAWLVKGRG (SEQ ID NO:37) |
| | HGEGTFTSDVSSYLEGQAAKEFIAWLVKGRG(Z) (SEQ ID NO:38) |
| | HGEGTFTSDVSSYLEGQAAK(B)EFIAWLVKGRG(Z) (SEQ ID NO:39) |
| Dulaglutide | HGEGTFTSDVSSYLEEQAAK(B)EFIAWLVKGGG (SEQ ID NO:40) |
| | HGEGTFTSDVSSYLEEQAAKEFIAWLVKGGG(Z) (SEQ ID NO:41) |
| | HGEGTFTSDVSSYLEEQAAK(B)EFIAWLVKGGG(Z) (SEQ ID NO:42) |
| Lixisenatide | HGEGTFTSDLSK(B)QMEEEAVRLFIEWLKNGGPSSGAPPSKK KKKK (SEQ ID NO:43) |
| | HGEGTFTSDLSKQMEEEAVRLFIEWLKNGGPSSGAPPSKK KKKK(Z) (SEO ID NO:44) |
| | HGEGTFTSDLSK(B)QMEEEAVRLFIEWLKNGGPSSGAPPSKK KKKK(Z) (SEQ ID NO:45) |
| Efpeglenatide | GEGTFTSDLSKQMEEEAVRLFIEWLK(B)NGGPSSGAPPPS (SEQ ID NO:46) |
| | GEGTFTSDLSKQMEEEAVRLFIEWLKNGGPSSGAPPPS(Z) (SEQ ID NO:47) |
| | GEGTFTSDLSKQMEEEAVRLFIEWLK(B)NGGPSSGAPPPS(Z) (SEQ ID NO:48) |
| Tirzepatide | YXEGTFTSDYSIXLDKIAQK(B)AFVQWLIAGGPSSGAPPPS (SEQ ID NO:49) |
| | YXEGTFTSDYSIXLDKIAQKAFVQWLIAGGPSSGAPPPS(Z) (SEQ ID NO:50) |
| | YXEGTFTSDYSIXLDKIAQK(B)AFVQWLIAGGPSSGAPPPS(Z) (SEQ ID NO:51) wherein each X represents α-aminoisobutyric acid (AIB) |

### PHARMACEUTICAL COMPOSITIONS

The compounds disclosed herein may be formulated as pharmaceutical compositions for administration by combination with a pharmaceutically-acceptable excipient, diluent, vehicle or carrier. In one embodiment, such compositions are prepared for oral administration. In other embodiments, the compound is prepared for parenteral administration, such as intravenous, subcutaneous or intramuscular administration. Further modes of administration are described below. In an embodiment, the compound is Compound I-A. In various embodiments, the compound is Compound I-B. In some embodiments, the compound is Compound I-C.

"Pharmaceutically acceptable carriers" include any excipient which is nontoxic to the cell or mammal being exposed thereto at the dosages and concentrations employed. The pharmaceutical composition may include one or more therapeutic agents, namely the compounds of the invention. Moreover, "pharmaceutically acceptable" refers to those compounds, materials, compositions, and/or dosage forms which are, within the scope of sound medical judgment, suitable for contact with the tissues of human beings and animals without excessive toxicity, irritation, allergic response, or other problem complications commensurate with a reasonable benefit/risk ratio. The term "pharmaceutically acceptable" also includes those carriers approved by a regulatory agency of the Federal or a state government or listed in the U.S. Pharmacopeia or other generally recognized pharmacopeia for use in animals and, more particularly, in humans.

Thus, as used herein, "pharmaceutically acceptable carrier" is intended to include any and all solvents, dispersion media, coatings, antibacterial and antifungal agents, isotonic and absorption delaying agents, and the like, compatible with pharmaceutical administration. Suitable carriers are described in the most recent edition of Remington's Pharmaceutical Sciences, a standard reference text in the field, which is incorporated herein by reference. Examples of such carriers or diluents include, but are not limited to, water, saline, Ringer's solutions, dextrose solution, and 5% human serum albumin. Liposomes and non-aqueous vehicles such as fixed oils may also be used. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

In an embodiment, pharmaceutical compositions comprising the therapeutic agent or agents, i.e., the compounds of the invention described herein, can be, in one embodiment, administered to a subject by any route known to a person skilled in the art, such as, without limitation, orally; parenterally, such as subcutaneously, intra-peritoneally, intravenously, intradermally, intramuscularly and intraarterially; transmucosally, such as transnasally, buccally, sublingually and rectally; intranasally; via inhalation; intra-vaginally; transdermally, intraosseously; intra-ventricularly, intra-cranially, epidurally, intrathecally or intra-tumorally. In certain embodiments, administration is orally.

Carriers may be any of those conventionally used, as described above, and are limited only by chemical-physical considerations, such as solubility and lack of reactivity with the compound of the invention, and by the route of administration. The choice of carrier will be determined by the particular route used to administer the pharmaceutical composition. Some examples of suitable carriers include lactose, glucose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water and methylcellulose. The formulations can additionally include lubricating agents such as talc, magnesium stearate, and mineral oil; wetting agents, surfactants, emulsifying and suspending agents; preserving agents such as methyl- and propylhydroxybenzoates; sweetening agents; flavoring agents, colorants, buffering agents (e.g., acetates, citrates or phosphates), disintegrating agents, moistening agents, antibacterial agents, antioxidants (e.g., ascorbic acid or sodium bisulfite), chelating agents (e.g., ethylenediaminetetraacetic acid), and agents for the adjustment of tonicity such as sodium chloride. Other pharmaceutical carriers can be sterile liquids, such as water and oils, including those of petroleum, animal, vegetable or synthetic origin, such as peanut oil, soybean oil, mineral oil, sesame oil and the like, polyethylene glycols, glycerine, propylene glycol or other synthetic solvents. In one embodiment, water, preferably bacteriostatic water, is the carrier when the pharmaceutical composition is administered intravenously or intratumorally. Saline solutions and aqueous dextrose and glycerol solutions can also be employed as liquid carriers, particularly for injectable solutions.

Pharmaceutical compositions suitable for injectable use may include sterile aqueous solutions (where water soluble) or dispersions and sterile powders for the extemporaneous preparation of sterile injectable solutions or dispersion. For intravenous administration, suitable carriers include, without limitation, physiological saline, bacteriostatic water, Cremophor EL.TM. (BASF, Parsippany, N.J.) or phosphate buffered saline (PBS). The composition should be sterile and should be fluid to the extent that easy syringeability exists. It should be stable under the conditions of manufacture and storage and be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol or sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as appropriate, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

The compositions and formulations as described herein may be administered alone or with other biologically-active agents. Administration can be systemic or local, e.g., through portal vein delivery to the liver. In addition, it may be advantageous to administer the composition into the central nervous system by any suitable route, including intraventricular and intrathecal injection. Intraventricular injection may be facilitated by an intraventricular catheter attached to a reservoir (e.g., an Ommaya reservoir). Pulmonary administration may also be employed by use of an inhaler or nebulizer, and formulation with an aerosolizing agent. It may also be desirable to administer the Therapeutic locally to the area in need of treatment; this may be achieved by, for example, and not by way of limitation, local infusion during surgery, topical application, by injection, by means of a catheter, by means of a suppository, or by means of an implant.

Pharmaceutically acceptable excipients, diluents, vehicles or carriers include components for making pills, tablets and the like for oral administration. Sterile aqueous solutions or dispersions and sterile powders may be provided for the extemporaneous preparation of sterile injectable solutions or dispersion. The use of such components for various routes of administration described herein, such as oral and parenteral administration of pharmaceutically active substances, is well known in the art.

In some embodiments, an oral formulation comprising a compound disclosed herein is enterically coated, to avoid exposure to gastric acids. Non-limiting examples of enteric coatings include polymers such as methyl acrylate-methacrylic acid copolymers, cellulose acetate phthalate (CAP), cellulose acetate succinate, hydroxypropyl methyl cellulose phthalate, hydroxypropyl methyl cellulose acetate succinate (hypromellose acetate succinate), polyvinyl acetate phthalate (PVAP), methyl methacrylate-methacrylic acid copolymers, shellac, cellulose acetate trimellitate, sodium alginate, zein, or any combination thereof.

In one aspect, the present invention provides a pharmaceutical composition comprising a compound comprising at least one G-protein coupled receptor (GPCR) agonist and/or antagonist covalently bound to at least one oral bioavailability enhancing moiety and/or protein binding enhancing moiety. In some embodiments, the at least one GPCR agonist and/or antagonist is a GLP-1 agonist or antagonist, a GIP agonist or antagonist, or a CRLR agonist or antagonist. In an embodiment, the at least one GPCR agonist is a GLP-1 agonist covalently bound to a GIP agonist. In certain embodiments, the at least one GPCR agonist and/or antagonist is a peptide. In some embodiments, each of the at least one oral bioavailability enhancing moiety and/or protein binding enhancing moiety is independently bound to an N-terminal amino acid residue, to a C-terminal amino acid residue, or to any non-terminal amino acid residue of the at least one GPCR agonist and/or antagonist, or introduced or replaced therein, or bound to any other component thereof. In an embodiment, the non-terminal amino acid residue is a lysine. In various embodiments, the at least one oral bioavailability enhancing moiety and/or protein binding enhancing moiety is covalently bound to the at least one GPCR agonist and/or antagonist by a linker. In some embodiments, the linker is polyethylene glycol. In certain embodiments, the at least one oral bioavailability enhancing moiety and/or protein binding enhancing moiety comprises a bile salt or bile acid. In an embodiment, the bile salt or bile acid is or comprises lithocholic acid, cholic acid, chenodeoxycholic acid, deoxycholic acid, ursodeoxycholic acid, or 7-ketolithocholic acid. In some embodiments, the at least one oral bioavailability enhancing moiety and/or protein binding enhancing moiety is or comprises a plurality of bile acids or bile salts which are covalently bound to the at least one GPCR agonist and/or antagonist by a linker. In an embodiment, the at least one GPCR agonist and/or antagonist has an N-terminal acetyl group, a C-terminal carboxamide group, or the combination thereof. In particular embodiments of the pharmaceutical composition, the at least one GPCR agonist is GLP-1, exenatide, liraglutide, albiglutide, dulaglutide, semaglutide, lixisenatide, efpeglenatide or tirzepatide. In some embodiments, the at least one GPCR agonist is semaglutide, and the at least one oral bioavailability enhancing moiety and/or protein binding enhancing moiety comprises lithocholic acid. In an embodiment, the moiety comprising lithocholic acid is bound to the semaglutide by a polyethylene glycol spacer. In certain embodiments, the polyethylene glycol bound to the lithocholic acid is bound to the C-terminal amino acid residue of the semaglutide. In an embodiment, the pharmaceutical composition comprises Compound I-A, as described herein, wherein n is between about 0 and 100. In some embodiments, n is about 10 to about 20. In an embodiment, the polyethylene glycol bound to the lithocholic acid moiety is bound to a non-terminal amino acid residue of the semaglutide. In some embodiments, the non-terminal amino acid residue is a lysine. In an embodiment, the pharmaceutical composition comprises Compound I- B, as described herein, wherein n is from about 0 to about 100. In an embodiment, n is about 10 to about 20. In an embodiment, the pharmaceutical composition comprises Compound I-C, as described herein, wherein each n is independently from 0 to 100. In some embodiments, each n is about 10 to about 20. In a particular embodiment of the pharmaceutical composition, the at least one GPCR agonist is tirzepatide, and the at least one oral bioavailability enhancing moiety and/or protein binding enhancing moiety comprises lithocholic acid. In some embodiments, the CRLR agonist is adrenomedullin, amylin or an analogue thereof. In various embodiments of the herein provided pharmaceutical compositions, the pharmaceutical composition comprises any one of the herein described compounds or combinations thereof and a pharmaceutically acceptable buffer, diluent, excipient, vehicle or carrier. In a particular embodiment, the pharmaceutical composition is formulated for oral administration. In some embodiments, the pharmaceutical composition is formulated for administration by any of the herein described administration routes.

As described herein, the compounds disclosed herein are orally bioavailable. In some embodiments, such compounds are administered parenterally. Such parenteral administration may allow for lower dose levels and/or less frequent administration, as a result of the increased protein binding by the compounds described herein.

### ROUTES OF ADMINISTRATION

The compounds disclosed herein may be formulated for administration by combination with a pharmaceutically-acceptable excipient, diluent, vehicle or carrier as described above, to prepare a pharmaceutical composition. In one embodiment, such pharmaceutical compositions are prepared for oral administration.

In some embodiments, pharmaceutical compositions are formulated for administration routes including but not limited to parenteral, such as subcutaneous, intra-peritoneal, intravenous, intra-dermal, intramuscular and intraarterial; transmucosal, such as trans-nasal, buccal, sublingual and rectal administration; intranasal administration; administration by inhalation; intra-vaginal administration; transdermal administration, intraosseous administration; intra-ventricular administration, intra-cranial administration, epidural administration, intrathecal administration or intra-tumoral administration. As disclosed herein, in one embodiment, oral administration is provided.

The dosing regimen, including but not limited to dose level, frequency of administration and duration of administration will be guided by the particular needs of the subject and prescribed by a health care professional. The dosing regimen will be readily established during clinical studies of the multimeric polypeptide for one or more diseases or conditions for which it is indicated.

### Effective doses

Furthermore, a skilled artisan would appreciate that the term "therapeutically effective amount" may encompass total amount of each active component of the pharmaceutical composition that is sufficient to show a meaningful patient benefit, i.e., treatment, healing, prevention or amelioration of the relevant medical condition, or an increase in rate of treatment, healing, prevention or amelioration of such conditions. When applied to an individual active ingredient, administered alone, the term refers to that ingredient alone. When applied to a combination, the term refers to combined amounts of the active ingredients that result in the therapeutic effect, whether administered in combination, serially or simultaneously.

Effective doses of the pharmaceutical compositions comprising the herein disclosed compounds of the present invention, for treatment of conditions and diseases, such as but not limited to, type 2 diabetes, obesity, insulin resistance, osteoporosis, migraine, Parkinson's disease, Alzheimer's disease, stroke, multiple sclerosis, traumatic brain injury and cardiovascular disease, vary depending upon many different factors, including means of administration, target site, physiological state of the patient, whether the patient is human or an animal, other medications administered, and whether treatment is prophylactic or therapeutic. In an embodiment, the pharmaceutical composition comprises a therapeutically effective amount of Compound I-A. In some embodiments, the pharmaceutical composition comprises a therapeutically effective amount of Compound I-B. In certain embodiments, the pharmaceutical composition comprises a therapeutically effective amount of Compound I-C.

Usually, the patient is a human, but non-human mammals including transgenic mammals can also be treated. Treatment dosages may be titrated using routine methods known to those of skill in the art to optimize safety and efficacy. The pharmaceutical compositions of the invention thus may include a "therapeutically effective amount" of the compounds described herein. A "therapeutically effective amount" refers to an amount effective, at dosages and for periods of time necessary, to achieve the desired therapeutic result. A therapeutically effective amount of a molecule may vary according to factors such as the disease state, age, sex, and weight of the individual, and the ability of the molecule to elicit a desired response in the individual. A therapeutically effective amount is also one in which any toxic or detrimental effects of the molecule are outweighed by the therapeutically beneficial effects.

Furthermore, a skilled artisan would appreciate that the term "therapeutically effective amount" may encompass a total amount of each active component (compounds described herein) of the pharmaceutical composition that is sufficient to show a meaningful patient benefit, i.e., treatment, healing, prevention or amelioration of the relevant medical condition, or an increase in rate of treatment, healing, prevention or amelioration of such conditions. When applied to an individual active ingredient, administered alone, the term refers to that ingredient alone. When applied to a combination, the term refers to combined amounts of the active ingredients that result in the therapeutic effect, whether administered in combination, serially or simultaneously.

The amount of a compound of the invention that will be effective in the treatment of a particular disorder or condition, such as but not limited to type 2 diabetes, obesity, insulin resistance, osteoporosis, migraine, Parkinson's disease, Alzheimer's disease, stroke, multiple sclerosis, traumatic brain injury and cardiovascular disease, also will depend on the nature of the disorder or condition, and can be determined by standard clinical techniques. In addition, *in vitro* assays may optionally be employed to help identify optimal dosage ranges. The precise dose to be employed in the formulation will also depend on the route of administration, and the seriousness of the disease or disorder, and should be decided according to the judgment of the practitioner and each patient's circumstances.

In one embodiment, the oral dose and dosing regimen of a compound disclosed herein may be guided by the dosing profile of GPCR agonists or antagonists without an oral bioavailability enhancing moiety and/or protein binding enhancing moiety, which are typically administered parenterally, such as subcutaneously, and typically administered weekly. By way of non-limiting example, a compound disclosed herein with an oral bioavailability of 10% may be administered at a dose of 10 times the parenteral dose, weekly. For ease in patient compliance for an oral medication, such dose may be adjusted to be effective by a single daily oral dose. Such guidance is merely exemplary as the health care professional with the oral bioavailability for a particular compound disclosed herein will readily identify the appropriate oral dose, depending on the frequency of administration. While non-limiting examples of such dose levels and dosing regimens are provided herein, such are merely exemplary and effective dose levels and dose regimens of compounds disclosed here are fully embraced herein.

In one embodiment, the dosage of the compound disclosed herein is within the range of about 0.001 to about 10 mg/kg of body weight. In another embodiment, the dosage will be within the range of about 0.001 mg/kg to about 1 mg/kg. In another embodiment, the dosage will be within the range of about 0.01 mg/kg to about 1 mg/kg. In another embodiment, the dosage will be within the range of about 0.001 mg/kg to about 0.1 mg/kg. In another embodiment, the dosage will be in the range of 0.1 mg/kg to about 10 mg/kg. In another embodiment, the dosage will be within the range of about 1 mg/kg to about 10 mg/kg. In an embodiment, the dosage is about 0.001 mg/kg. In another embodiment, the dosage is 0.01 mg/kg.

In an embodiment, the pharmaceutical composition comprising a compound disclosed herein is administered orally at a dose of 0.001 mg, 0.005 mg, 0.010 mg, 0.1 mg, 0.5 mg, 1.0 mg 2.5 mg, 5 mg, 7.5 mg, 10 mg, 12.5 mg, 15 mg, 20 mg, 25 mg, 30 mg, 35 mg, 40 mg, 45 mg, or 50 mg. In a particular embodiment, the pharmaceutical composition comprising a compound disclosed herein is formulated for oral administration. In an embodiment, the pharmaceutical composition comprising a compound disclosed herein is administered orally as a capsule or tablet in a dose based on body weight of the subject and on the above-described factors. In certain embodiments, the orally administered pharmaceutical composition comprising a compound disclosed herein is taken (i.e., is administered) as a single dose; however, a physician may adjust dose, as needed.

In some embodiments, the pharmaceutical composition comprising a compound disclosed herein is formulated for parenteral injections, including intradermal (IM), subcutaneous (SQ), intramuscular (IM), and intravenous (IV) administration. In some embodiments, such parenteral administration may be less frequent that compounds without a protein binding enhancing moiety because of increased half-life conferred by the moiety.

In particular embodiments, the pharmaceutical composition comprises a therapeutically effective amount of Compound I-A, Compound I-B, Compound I-C or combinations thereof. In particular embodiments, the oral pharmaceutical composition comprises a therapeutically effective amount of Compound I-A, Compound I-B, Compound I-C or combinations thereof.

The administration and dose of the orally administered pharmaceutical composition comprising a compound disclosed herein for a child subject is determined by the physician.

The herein described compound(s) or the pharmaceutical composition(s) comprising said compounds may be administered only once daily, or it may be administered multiple times. For multiple dosages, the pharmaceutical composition may, for example, be administered three times a day, twice a day, once a day, once every two days, twice a week, weekly, once every two weeks, or monthly.

### MEDICAL USES

In one aspect, the present invention provides a compound of the invention or a pharmaceutical composition of the invention, as disclosed herein, for use as a medicament. The present invention also provides a compound of the invention or a pharmaceutical composition of the invention, as disclosed herein, for use in treating a GPCR agonist and/or antagonist responsive condition or disease in a subject. Thus, the present invention provides a compound comprising at least one G-protein coupled receptor (GPCR) agonist and/or antagonist covalently bound to at least one oral bioavailability enhancing moiety and/or protein binding enhancing moiety or a pharmaceutical composition comprising the compound, as described herein, for use in treating a GPCR agonist and/or antagonist responsive condition or disease in a subject in need thereof by administering the compound or the pharmaceutical composition to the subject. In a particular embodiment, administration of the herein described compound or the herein described pharmaceutical composition comprising the said compound is oral. In an embodiment, the circulating half-life and/or the exposure of the compound is increased.

Compounds and pharmaceutical compositions disclosed herein may be used for treating any GPCR agonist or antagonist responsive condition or disease by administering to a subject in need thereof a therapeutically effective amount of a compound as described hereinabove or by administering a pharmaceutical composition comprising any compound as described herein in a therapeutically effective amount of said compound. In some embodiments, the administration is oral.

Compounds disclosed herein are in some embodiments and in particular embodiments wherein the compound comprises at least one GPCR agonist, useful in the treatment of obesity, type 2 diabetes, osteoporosis, lymphatic insufficiency, migraine or cardiovascular disease, among others. In some embodiments, compounds disclosed herein are in some embodiments and in particular, wherein the compound comprises at least one GPCR antagonist, useful in the treatment of excessive hormone activity, manipulation of the reproductive endocrine axis, or treatment of cancer. In an embodiment, the pharmaceutical composition comprises a therapeutically effective amount of Compound I-A. In some embodiments, the pharmaceutical composition comprises a therapeutically effective amount of Compound I-B. In certain embodiments, the pharmaceutical composition comprises a therapeutically effective amount of Compound I-C. In an embodiment, the pharmaceutical composition comprises a therapeutically effective amount of a combination of Compound I-A, Compound I-B and/or Compound I-C.

### Definitions

As employed above and throughout the disclosure, the following terms and abbreviations, unless otherwise indicated, shall be understood to have the following meanings.

The terms "comprise", "comprises", "comprising", "includes", "including", "having" and their conjugates mean "including but not limited to".

Further, unless otherwise required by context, singular terms shall include pluralities and plural terms shall include the singular. As used herein, the singular form "a", "an" and "the" include plural references unless the context clearly dictates otherwise. For example, the term "an enzyme" or "at least one enzyme" may include a plurality of enzymes, including mixtures thereof.

Throughout this application, various embodiments of the present disclosure may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range. In some embodiments, plurality refers to more than one and comprises any of the foregoing values.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween. All ranges are inclusive and combinable.

In some embodiments, a value of "about" is meant to be within 1%, 2%, 3%, 4%, 5%, 6%, 7%, 8%, 9%, 10%, 11%, 12%, 13%, 14%, 15%, 16%, 17%, 18%, 19%, or 20% of the value.

As used herein, the terms "component," "composition," "composition of compounds," "compound," "drug," "pharmacologically active agent," "active agent," "therapeutic," "therapy," "treatment," or "medicament" are used interchangeably herein to refer to a compound or compounds or composition of matter described herein, when administered to a subject (human or animal) induces a desired pharmacological and/or physiologic effect by local and/or systemic action.

As used herein, the terms "treatment" or "therapy" (as well as different forms thereof) include preventative (e.g., prophylactic), curative or palliative treatment. As used herein, the term "treating" includes alleviating or reducing at least one adverse or negative effect or symptom of a condition, disease or disorder.

The terms "subject," "individual," and "patient" are used interchangeably herein, and refer to an animal, such as a human and non-human animals, to whom treatment is administered, including prophylactic treatment, with the pharmaceutical compositions according to the present invention. The terms "non-human animals" and "non-human mammals" are used interchangeably herein and include all vertebrates, e.g., mammals, such as non-human primates, (particularly higher primates), sheep, dog, rodent, (e.g., mouse or rat), guinea pig, goat, pig, cat, rabbits, cows, horses and non-mammals such as reptiles, amphibians, chickens, and turkeys.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting. Each literature reference or other citation referred to herein is incorporated herein by reference in its entirety.

In the description presented herein, each of the steps of the invention and variations thereof are described. This description is not intended to be limiting and changes in the components, sequence of steps, and other variations would be understood to be within the scope of the present invention.

### EXAMPLES

### Example 1. Preparation of Compounds

Compounds described herein are made by linking, for example, a GLP-1 agonist such as semaglutide to one or more oral bioavailability enhancing moieties and/or protein binding enhancing moieties such as a moiety comprising lithocholic acid. Examples of such moieties include (B) and (Z) as described herein. In one example, Compound I-A is made by conjugating moiety (Z) to the C-terminus of semaglutide. In one example, Compound I-B is made by conjugating moiety (B) to the lysine at position 20 of semaglutide in place of the lipid. In one example, Compound I-C is made by conjugating moiety (B) to lysine 20 of semaglutide in place of the lipid, and also conjugating moiety (Z) to the C terminus. Guidance on such conjugation may be found herein and is generally known in the art. Once prepared, compounds can be isolated and/or purified by methods well known in peptide purification methods, such as to a purity of at least 99%. Such purified compounds may be formulated into a solid dosage form for oral administration.

### Example 2. Treatment of Obesity

A patient presents with obesity. The patient is started on a weekly oral dose of a pharmaceutical composition comprising Compound I-A in a enterically-coated tablet. A reduction in weight is observed over time.

### Example 3. Treatment of Diabetes

A patient presents with type 2 diabetes. The patient is started on a daily oral dose of a pharmaceutical composition comprising Compound I-B. Improvement in diabetic symptoms is overserved over time.

### Example 4. Treatment of Metabolic Syndrome

A patient presents with metabolic syndrome. The patients is started on a daily oral dose of a pharmaceutical composition of Compound I-C. Improvement in metabolic syndrome is overserved over time.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Having described certain embodiments of the present invention, it is to be understood that the invention is not limited to the precise embodiments, and that various changes and modifications may be effected therein by those skilled in the art without departing from the scope or spirit of the invention as defined in the appended claims.

## Claims

1. A compound comprising at least one G-protein coupled receptor (GPCR) agonist and/or antagonist covalently bound to at least one oral bioavailability enhancing moiety and/or protein binding enhancing moiety.

2. The compound of claim 1, wherein the at least one GPCR agonist and/or antagonist is a GLP-1 agonist or antagonist; a GIP agonist or antagonist; a CRLR agonist or antagonist, such as adrenomedullin, amylin or an analogue thereof; or a GLP-1 agonist covalently bound to a GIP agonist.

3. The compound of any of claims 1 or 2, wherein the at least one GPCR agonist and/or antagonist is a peptide, such as exenatide, liraglutide, albiglutide, dulaglutide, semaglutide, lixisenatide, efpeglenatide or tirzepatide.

4. The compound of any of claims 1-3, wherein each of the at least one oral bioavailability enhancing moiety and/or protein binding enhancing moiety is independently bound to an N-terminal amino acid residue, to a C-terminal amino acid residue, or to any non-terminal amino acid residue of the at least one GPCR agonist and/or antagonist, such as a lysine residue, or introduced or replaced therein, or bound to any other component thereof.

5. The compound of any of claims 1-4, wherein the at least one oral bioavailability enhancing moiety and/or protein binding enhancing moiety comprises a bile salt or bile acid such as lithocholic acid, cholic acid, chenodeoxycholic acid, deoxycholic acid, ursodeoxycholic acid, or 7-ketolithocholic acid, optionally wherein a plurality of bile acids or bile salts are covalently bound.

6. The compound of any of claims 1-5, wherein the at least one GPCR agonist and/or antagonist has an N-terminal acetyl group, a C-terminal carboxamide group, or the combination thereof.

7. The compound of any of claims 1-6, wherein the oral bioavailability enhancing moiety and/or protein binding enhancing moiety is (B) or (Z): 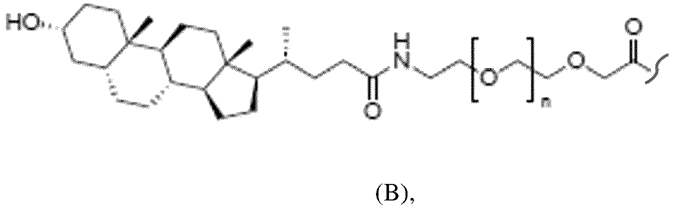 wherein n is between 0 and about 100; 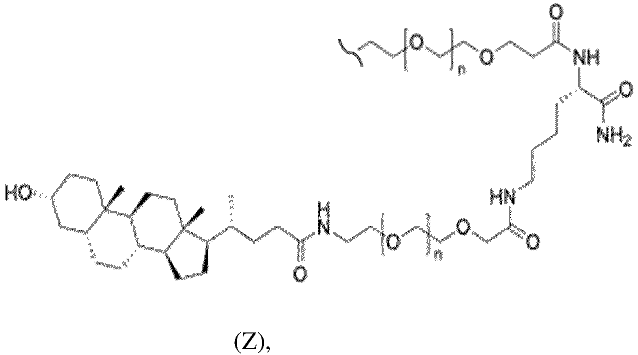 wherein each n is independently between 0 and about 100.

8. The compound of claim 7, wherein the GPCR agonist is semaglutide and the oral bioavailability enhancing moiety and/or protein binding enhancing moiety comprises lithocholic acid, and the oral bioavailability enhancing moiety and/or protein binding enhancing moiety is (B) or (Z): 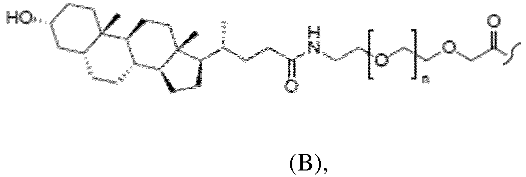 wherein n is between 0 and about 100; 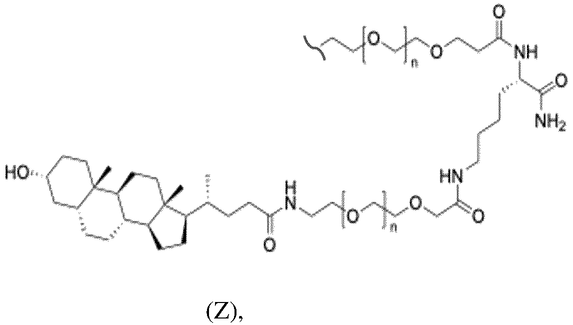 wherein each n is independently between 0 and about 100.

9. The compound of claim 8, wherein (Z) is bound to the C terminus of semaglutide, for example wherein the compound is Compound (I-A) 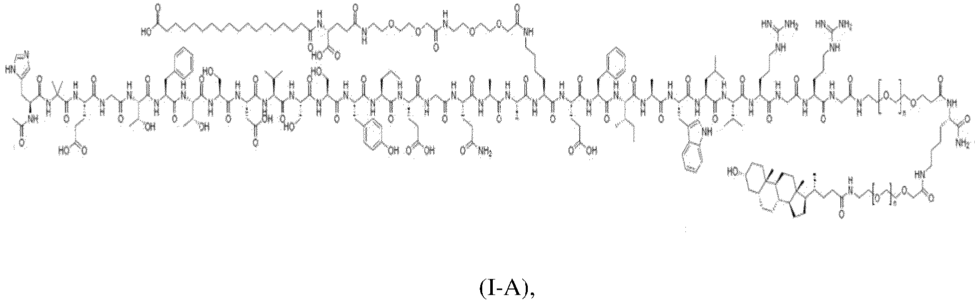 wherein each n is independently between 0 and about 100.

10. The compound of claim 8, wherein the moiety comprising the lithocholic acid is bound to a non-terminal amino acid of semaglutide, such as a lysine, for example wherein the compound is Compound (I-B) 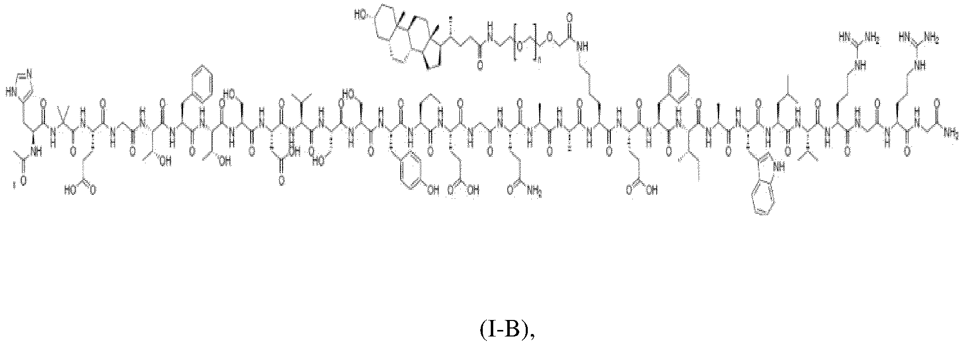 wherein n is from 0 to about 100.

11. The compound of claim 8, wherein the moiety comprising the lithocholic acid is bound to a non-terminal amino acid and to the C-terminal amino acid of semaglutide, for example wherein the compound is Compound (I-C) 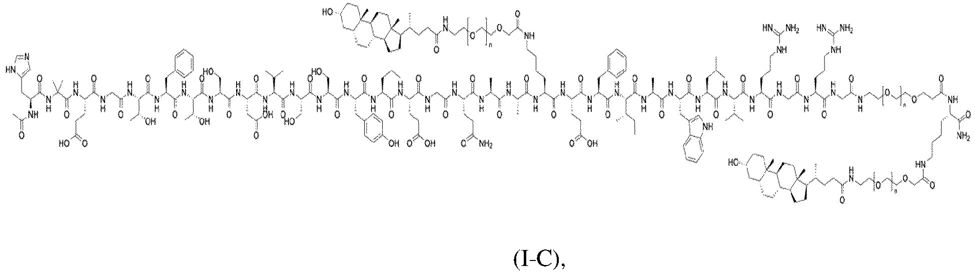 wherein each n is independently from 0 to 100.

12. The compound of any of claims 7-11, wherein each n is independently about 10 to about 20, for example wherein each n is independently 2, 4, 8 or 12.

13. The compound of any of claims 1-7, wherein the at least one GPCR agonist is tirzepatide, and the at least one oral bioavailability enhancing moiety and/or protein binding enhancing moiety comprises lithocholic acid.

14. A pharmaceutical composition comprising the compound of any of claims 1-13 and a pharmaceutically acceptable buffer, diluent, excipient, vehicle or carrier, optionally formulated for oral administration.

15. The compound of any of claims 1-13 or the pharmaceutical composition of claim 14 for use in treating a GPCR agonist and/or antagonist responsive condition or disease in a subject in need thereof, optionally by oral administration, wherein the GPCR agonist and/or antagonist responsive condition or disease is type 2 diabetes, obesity, insulin resistance, osteoporosis, migraine, Parkinson's disease, Alzheimer's disease, stroke, multiple sclerosis, traumatic brain injury, cardiovascular disease, hormone imbalance, reproductive endocrine axis dysfunction, or cancer.
